(19) **Europäisches Patentamt**
**European Patent Office**
**Office européen des brevets**

(11) **EP 3 632 322 B1**

(12) **EUROPEAN PATENT SPECIFICATION**

(45) Date of publication and mention
of the grant of the patent:
**13.08.2025 Bulletin 2025/33**

(21) Application number: **18809417.1**

(22) Date of filing: **04.06.2018**

(51) International Patent Classification (IPC):
*A61B 5/024* (2006.01)     *A61B 5/00* (2006.01)
*A61B 5/0245* (2006.01)     *G16H 50/70* (2018.01)

(52) Cooperative Patent Classification (CPC):
**A61B 5/02405; A61B 5/4812; A61B 5/7267;**
A61B 5/0022; A61B 5/0245; A61B 5/7246;
G16H 50/70

(86) International application number:
**PCT/JP2018/021420**

(87) International publication number:
**WO 2018/221750 (06.12.2018 Gazette 2018/49)**

(54) **SLEEP DETERMINING DEVICE, SLEEP DETERMINING METHOD, AND SLEEP DETERMINING PROGRAM**

SCHLAFBESTIMMUNGSVORRICHTUNG, SCHLAFBESTIMMUNGSVERFAHREN UND
SCHLAFBESTIMMUNGSPROGRAMM

DISPOSITIF, PROCÉDÉ ET PROGRAMME DE DÉTERMINATION DU SOMMEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2017 JP 2017110495**

(43) Date of publication of application:
**08.04.2020 Bulletin 2020/15**

(73) Proprietor: **e-Life Co. Ltd.**
**Tokyo 105-0003 (JP)**

(72) Inventors:
• **MITSUKURA, Yasue**
**Yokohama-shi**
**Kanagawa 223-8522 (JP)**
• **YASUI, Masato**
**Tokyo 160-8582 (JP)**
• **FUKUNAGA Koichi**
**Tokyo 160-8582 (JP)**
• **FURUKAWA Toshiharu**
**Tokyo 160-8582 (JP)**

(74) Representative: **Grünecker Patent- und**
**Rechtsanwälte**
**PartG mbB**
**Leopoldstraße 4**
**80802 München (DE)**

(56) References cited:
WO-A1-2018/042566     JP-A- 2012 065 853
JP-A- 2013 202 121     JP-A- 2016 014 542
US-A1- 2015 190 086     US-A1- 2015 190 086
US-A1- 2016 007 868     US-A1- 2016 310 696

• **SHANSHAN YU ET AL: "Automatic sleep stage
classification based on ECG and EEG features
for day time short nap evaluation", INTELLIGENT
CONTROL AND AUTOMATION (WCICA), 2012
10TH WORLD CONGRESS ON, IEEE, 6 July 2012
(2012-07-06), pages 4974 - 4977, XP032270336,
ISBN: 978-1-4673-1397-1, DOI: 10.1109/
WCICA.2012.6359421**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a sleep determination apparatus, a sleep determination method, and a sleep determination program.

BACKGROUND ART

In the related art, a sleep polysomnography (PSG) test based on an electroencephalogram, a respiratory test, an eye movement test, an electrocardiogram, and a blood pressure test, and other test items is known to determine a sleep stage.

However, the PSG test involves measurement in a facility, such as a hospital, such that a PSG device having a large number of electrodes is attached to the body of a user, and imposes constraints such as heavy physical and mental loads. Another issue is that such a PSG test needs to be conducted by a skilled expert to determine a sleep stage from the test items obtained using the PSG device, which incurs a large cost.

Accordingly, methods have been devised in recent years for easily estimating a sleep stage, without using a PSG device, from a heart rate signal, electrocardiogram information, and so on obtained using a wristwatch-type measurement device or the like (see NPLs 1, 2, and 3).

Citation List

Non Patent Literature

NPL 1: SM Isa, I Wasito, "Kernel dimensionality reduction on sleep stage classification using ECG signal", IJCSI International Journal of Computer Science Issues, Vol. 8, Issue 4, No 2, July 2011
NPL 2: K Tanida, M Shibata, "Sleep stage assessment using power spectral indices of heart rate variability with a simple algorithm: limitations clarified from preliminary study", Biological Research for Nursing, Vol. 15, Issue 3, 2013
NPL 3: P Fonseca, X Long, M Radha, R Haakma, R Aarts and J Rolink, "Sleep stage classification with ECG and respiratory effort", Physiological Measurement, Volume 36, Number 10

US 2015/190086 A1 relates to a system for automatic sleep staging, wherein sleep stages including a single light sleep non-REM stage are determines using heart rata data, accelerometer readings, skin temperature signals and respiratory signals are utilized.

Shanshan Yu et al: "Automatic sleep stage classification based on ECG and EEG features for day time short nap evaluation", 10th World Congress on Intelligent Control and Automation (WCICA), July 6, 2020, pages 4974-4977 relates to automatic sleep stage classification using features derived from a ECG data in combination with EEG data.

US 2016/310696 A1 relates to a system for sleep stage classification making use of cardiac and respiratory attributes to classify sleep stages including a single light speed non-REM stage.

However, the sleep stage estimation methods of the related art can determine only four out of five sleep stages including the REM sleep stage and first to fourth non-REM sleep stages. In addition, the maximum precision in the determination of the four sleep stages is about 0.69 (69%). Accordingly, there is an issue of insufficient accuracy.

The present invention has been made in view of the foregoing issues, and provides a sleep determination apparatus, a sleep determination method, and a sleep determination program that provide an accurate and detailed determination of a sleep stage.

The invention is defined by the independent claims. The dependent claims describe advantageous embodiments.

According to the present invention, it can be possible to perform an accurate and detailed determination of a sleep stage.

BRIEF DESCRIPTION OF THE DRAWINGS

Fig. 1 is a block diagram illustrating an example configuration of the sleep determination apparatus 100 to which this embodiment is applied.

Fig. 2 is a flowchart illustrating an example of a sleep determination process performed by the sleep determination apparatus 100 according to this embodiment.

Fig. 3 is a diagram schematically illustrating, as a learning algorithm, an ensemble learning technique using decision trees in a random forest (RF).

Fig. 4 is a graph chart illustrating a relationship of changes in prediction error with respect to the number of nodes and the number of decision trees in the case of permutation of explanatory variables.

Fig. 5 is a graph chart illustrating the importance of the explanatory variables determined using the equation described above.

Fig. 6 is a diagram illustrating a relationship between the number of nodes and the prediction error in a random forest.

Fig. 7 is conceptual diagram schematically illustrating a recurrent neural network (RNN).

Fig. 8 is conceptual diagram schematically illustrating a recurrent neural network (RNN).

Fig. 9 is a diagram illustrating an example of the result of R-wave detection.

Fig. 10 is a diagram illustrating an example of the RRI time-series data.

Fig. 11 is a diagram illustrating PSD data computed from the RRI.

Fig. 12 is a diagram illustrating the RRI in each sleep stage.

Fig. 13 is a diagram illustrating the standard deviation in each sleep stage.

Fig. 14 is a diagram illustrating the LF in each sleep stage.

Fig. 15 is a diagram illustrating the VLF in each sleep stage.

Fig. 16 is a diagram illustrating the HF in each sleep stage.

Fig. 17 is a diagram illustrating the LF/HF ratio in each sleep stage.

Fig. 18 is a diagram illustrating a relationship between the number of layers in the RNN and changes in recognition rate.

Fig. 19 is a diagram illustrating a relationship between the number of clusters in the HMM and changes in recognition rate.

PREFERRED MODE FOR CARRYING OUT THE INVENTION

In the following, a sleep determination apparatus, a sleep determination method, and a sleep determination program according to this embodiment of the present invention, and an embodiment of a recording medium will be described in detail with reference to the drawings. It should be noted that this embodiment is not intended to limit the present invention.

[Configuration of Sleep Determination Apparatus 100]

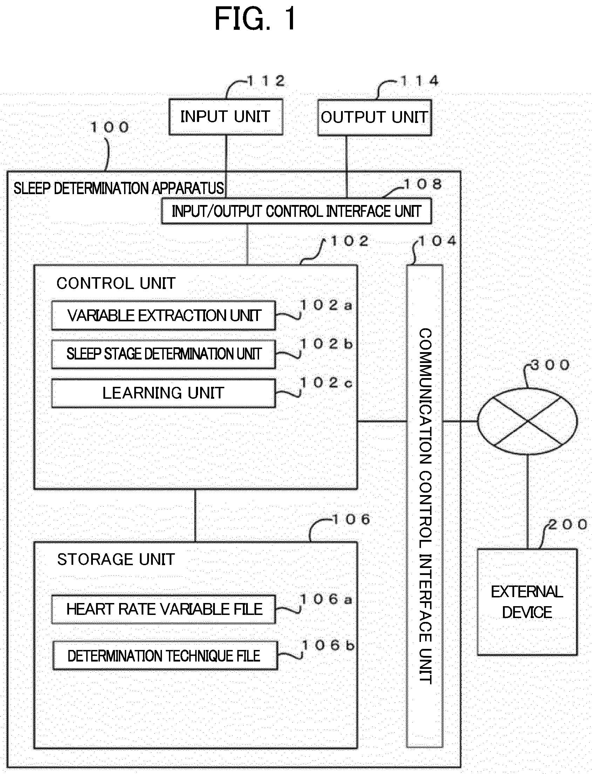

First, a configuration of a sleep determination apparatus 100 according to this embodiment will be described, and, then, processing and the like in a sleep determination method according to this embodiment will be described in detail. Fig. 1 is a block diagram illustrating an example configuration of the sleep determination apparatus 100 to which this embodiment is applied. In the block diagram, only a portion of the illustrated configuration, which is related to this embodiment, is conceptually illustrated. The sleep determination apparatus 100 may employ a device configuration of a personal computer or a device configuration of a mobile terminal such as a smartphone or a wearable terminal, or may be a server apparatus.

In Fig. 1, the sleep determination apparatus 100 generally includes a control unit 102 such as a CPU that collectively controls the overall operation of the sleep determination apparatus 100, a communication control interface unit 104 to be connected to a communication device such as a router (not illustrated) to be connected to a communication line or the like, an input/output control interface unit 108 to be connected to an input unit 112 or an output unit 114, and a storage unit 106 that stores various databases, tables, and so on. These components are communicably connected to each other via any desired communication path.

The various databases and tables (such as a heart rate variable file 106a and a determination technique file 106b) stored in the storage unit 106 are storage means, such as a fixed disk device, and configured to store various programs, tables, files, databases, web pages, and so on that are used for various processing operations.

Among them, the heart rate variable file 106a is a heart rate variability parameter storage means for storing heart rate variability parameters. As an example, the heart rate variable file 106a may store the very low frequency component (VLF), low frequency component (LF), and high frequency component (HF) of heart rate variability, the mean heartbeat interval (RRI), the standard deviation of each heartbeat interval (RRI), the ratio of the low frequency component to the high frequency component (LF/HF ratio), and so on as heart rate variability parameters.

The determination technique file 106b is a determination technique storage means for storing a determination technique for determining a sleep stage using heart rate variability parameters (e.g., VLF, LF, HF, mean RRI, standard deviation of each RRI, and LF/HF ratio). For example, the determination technique file 106b may store a mathematical formula, an algorithm, or the like that takes heart rate variability parameters as input values and that outputs evaluation values of sleep stages. As a more specific example, the determination technique file 106b may store algorithm data or the like regarding learning results that are obtained by learning, from training data for which sleep stages are known, using the heart rate variability parameters as inputs.

Alternatively, the storage unit 106 may store heart rate variability data detected from the user. For example, the storage unit 106 may store heart rate variability data input from the input unit 112 or heart rate variability data transmitted from an external device 200. Further, the storage unit 106 may store training data for which sleep stages are known. More specifically, the storage unit 106 may store a dataset that associates a sleep stage determined by an expert using a PSG test with heart rate variability parameters set at that time. The storage unit 106 may also store a result of determining a sleep stage by a sleep stage determination unit 102b described below.

In Fig. 1, furthermore, the input/output control interface unit 108 controls the input unit 112 and the output unit 114. As an example, in this embodiment, the input unit 112 may be an input means for inputting heart rate signal data. For example, the input/output control interface unit 108 may be a heart rate detection means capable of detecting a heart rate signal, such as a pressure detection sensor or a vibration detection sensor. The input unit 112 need not be physically connected to the sleep determination apparatus 100. The input unit 112 may be connected to the sleep determination apparatus 100 as a heart rate signal detection device to be attached to a user, such as a wearable terminal, via a wireless communication means such as Bluetooth (registered trademark). Alternatively, the input unit 112 may be an input terminal (e.g., a USB port) or the like for an external storage medium such as a USB memory storing heart rate signal data and so on. Other examples of the input unit 112 include a keyboard, a mouse, and a touch panel. The output unit 114 may be an output means such as a monitor (including a home TV monitor, a touch screen monitor, or the like), or may be a printing means such as a printer.

In Fig. 1, furthermore, the control unit 102 has an internal memory for storing a control program such as an OS (Operating System), a program defining various processing procedures and the like, and required data, and is configured to perform information processing using these programs and the like to execute a variety of processing operations. The control unit 102 functionally and conceptually includes a variable extraction unit 102a, a sleep stage determination unit 102b, and a learning unit 102c.

Among them, the variable extraction unit 102a is a heart rate data obtaining means for obtaining a heart rate variability parameter indicating a heart rate state from the heart rate variability data stored in the storage unit 106. For example, the variable extraction unit 102a may extract the very low frequency component (VLF), low frequency component (LF), and high frequency component (HF) of heart rate variability, the mean heartbeat interval (RRI), the standard deviation of each heartbeat interval (RRI), the ratio of the low frequency component to the high frequency component (LF/HF ratio), and so on as heart rate variability parameters.

As an example, the variable extraction unit 102a may filter the heart rate signal data stored in the storage unit 106 to remove noise, and detect a peak interval signal from a filtered heart rate signal. Further, the variable extraction unit 102a may calculate a power spectral density from the peak interval signal and calculate the HF value, the LF value, the HF/LF ratio, the mean or standard deviation of each RRI, and so on from the calculated power spectral density. More specifically, the peak interval signal is a signal having a variable that is an interval between waveforms (R-waves) around peak intensities of a heart rate signal. In heart rate variability analysis, the peak interval signal is known as an R-R interval (RRI) signal indicating an interval between adjacent R-wave peaks. The variable extraction unit 102a may calculate the mean or standard deviation of each RRI on the basis of the peak interval (RRI). As an example, the variable extraction unit 102a performs frequency analysis, such as Fourier transform, on the detected peak interval signal to calculate a power spectral density. The power spectral density shows a different distribution depending on the state of the autonomic nervous system. In this embodiment, the VLF value is a local maximum value in a range less than about 0.04 Hz, the LF value is a local maximum value in a range of about 0.04 Hz to 0.15 Hz, and the HF value is a local maximum value in a range of about 0.15 Hz to 0.40 Hz. The variable extraction unit 102a can calculate the LF/HF ratio from the determined LF and HF values. Alternatively, the variable extraction unit 102a may extract the heart rate variability parameters from the heart rate variability data by using a known parameter extraction technique.

The sleep stage determination unit 102b is a sleep stage determining means for determining a sleep stage using the heart rate variability parameters extracted by the variable extraction unit 102a. As an example, the sleep stage determination unit 102b may determine light sleep among the sleep stages. For example, the sleep stage determination unit 102b may determine a light sleep stage including first and second non-REM sleep stages (N1 & N2), which is not covered by the present invention, or, according to the present invention, the sleep stage determination unit 102b is configured to determine the first non-REM sleep stage (N1) or the second non-REM sleep stage (N2) by distinguishing in detail between the first non-REM sleep stage (N1) and the second non-REM sleep stage (N2). Further, the sleep stage determination unit 102b is configured to determine a sleep stage by distinguishing, as sleep stages, five stages, including the REM sleep stage (REM), the first non-REM sleep stage (N1), the second non-REM sleep stage (N2), the third non-REM sleep stage (N3), and the fourth non-REM sleep stage (N4). Alternatively, in an example not part of the present invention, the sleep stage determination unit 102b may determine a sleep stage by distinguishing, as sleep stages, four stages, including the REM sleep stage (REM), the light sleep stage including the first and second non-REM sleep stages (N1 & N2), the third non-REM sleep stage (N3), and the fourth non-REM sleep stage (N4).

The sleep stage determination unit 102b may determine a sleep stage by calculating evaluation values or the like of the sleep stages from the heart rate variability parameters using the mathematical formula or algorithm stored in the determination technique file 106b. For example, the sleep stage determination unit 102b may obtain, from the storage unit 106, learning result data that is obtained by learning, from training data for which sleep stages are known, using the heart rate variability parameters as inputs, and determine a sleep stage on the basis of the obtained learning result data.

Alternatively, the sleep stage determination unit 102b may calculate a parameter indicating which of the sleep stages (N1 to N4 and REM) including the first non-REM sleep stage (N1) and the second non-REM sleep stage (N2) the heart rate variability parameters to be used to determine a sleep stage indicate, and determine a sleep stage identified by the parameter with high accuracy. For example, the sleep stage determination unit 102b may calculate the probability $\sigma rem(\tau)$ of being in the REM sleep stage (REM) at time $\tau$, the probability $\sigma n1(\tau)$ of being in the first non-REM sleep stage (N1) at the time $\tau$, the probability $\sigma n2(\tau)$ of being in the second non-REM sleep stage (N2) at the time $\tau$, the probability $\sigma n3(\tau)$ of being in the third non-REM sleep stage (N3) at the time $\tau$, and the probability $\sigma n4(\tau)$ of being in the fourth non-REM sleep stage (N4) at the time $\tau$ using Eqs. (1) to (5) below, and determine that a sleep stage indicated by the heart rate variability parameters is the sleep stage having the highest probability.

[Math. 1]

$$\sigma_{rem}(\tau) = \frac{1}{1+e^{-(-a1-b1\cdot[RR(\tau)]+c1\cdot[RR_{std}(\tau)]-d1\cdot[VLF(\tau)]-e1\cdot[LF(\tau)/HF(\tau)])}} \quad (1)$$

$$\sigma_{n1}(\tau) = \frac{1}{1+e^{-(-a2-b2\cdot[RR(\tau)]+c2\cdot[RR_{std}(\tau)]-d2\cdot[VLF(\tau)]-e2\cdot[LF(\tau)/HF(\tau)])}} \quad (2)$$

$$\sigma_{n2}(\tau) = \frac{1}{1+e^{-(-a3-b3\cdot[RR(\tau)]+c3\cdot[RR_{std}(\tau)]-d3\cdot[VLF(\tau)]-e3\cdot[LF(\tau)/HF(\tau)])}} \quad (3)$$

$$\sigma_{n3}(\tau) = \frac{1}{1+e^{-(-a4-b4\cdot[RR(\tau)]+c4\cdot[RR_{std}(\tau)]-d4\cdot[VLF(\tau)]-e4\cdot[LF(\tau)/HF(\tau)])}} \quad (4)$$

$$\sigma_{n4}(\tau) = \frac{1}{1+e^{-(-a5-b5\cdot[RR(\tau)]+c5\cdot[RR_{std}(\tau)]-d5\cdot[VLF(\tau)]-e5\cdot[LF(\tau)/HF(\tau)])}} \quad (5)$$

In Eqs. (1) to (5), a1 to a5, b1 to b5, c1 to c5, d1 to d5, and e1 to e5 are constants and may be statistically determined from measurement data of a plurality of subjects or determined from the result of determining measurement data of an individual subject by an expert using PSG. Further, RR($\tau$) denotes the mean heartbeat interval at the time $\tau$, RRstd($\tau$) denotes the standard deviation of the heartbeat interval at the time $\tau$, VLF($\tau$) denotes the very low frequency component of heart rate variability at the time $\tau$, LF($\tau$) denotes the low frequency component of heart rate variability at the time $\tau$, and HF($\tau$) denotes the high frequency component of heart rate variability at the time $\tau$. In the calculation of the probability $\sigma rem(\tau)$ of being in the REM sleep stage (REM) at the time $\tau$, the probability $\sigma n1(\tau)$ of being in the first non-REM sleep stage (N1) at the time $\tau$, the probability $\sigma n2(\tau)$ of being in the second non-REM sleep stage (N2) at the time $\tau$, the probability $\sigma n3(\tau)$ of being in the third non-REM sleep stage (N3) at the time $\tau$, and the probability $\sigma n4(\tau)$ of being in the fourth non-REM sleep stage (N4) at the time $\tau$, the respective probabilities may be calculated in such a manner as to reflect data obtained a predetermined time ago.

In this embodiment, furthermore, the first non-REM sleep stage (N1) and the second non-REM sleep stage (N2) are distinguished and determined, the determination being based on a first determination condition that is set based on a correlation between the heart rate variability parameters and the first non-REM sleep stage (N1) and a second determination condition that is set based on a correlation between the heart rate variability parameters and the second non-REM sleep stage (N2). For example, the following determination technique may be used.

Specifically, threshold values th1 to th4, each for distinguishing between the first non-REM sleep stage (N1) and the second non-REM sleep stage (N2), are set for the very low frequency component (VLF) of heart rate variability, the ratio of the low frequency component (LF) to the high frequency component (HF) (LF/HF ratio), the mean heartbeat interval (RRI), and the standard deviation of each heartbeat interval (RRI), respectively. The threshold values th1 to th4 may be statistically determined from measurement data of a plurality of subjects or determined from the result of determining measurement data of an individual subject by an expert using PSG.

Then, for the heart rate variability parameters to be used to determine a sleep stage, the condition for the respective threshold values th1 to th4 (whether the heart rate variability parameters are larger than the threshold values or smaller than the threshold values) is determined.

For example, if the respective values of the very low frequency component (VLF) of heart rate variability, the ratio of the low frequency component (LF) to the high frequency component (HF) (LF/HF ratio), and the standard deviation of each heartbeat interval (RRI) are larger than the set threshold values th1, th2, and th4, respectively, the first non-REM sleep stage (N1) may be determined. If the value of the mean heartbeat interval (RRI) is smaller than the set threshold value th3, the first non-REM sleep stage (N1) may be determined.

From the determination results described above, it can be determined which of the first non-REM sleep stage (N1) and the second non-REM sleep stage (N2) the heart rate variability parameters to be used to determine a sleep stage indicate. For example, which of the first non-REM sleep stage (N1) and the second non-REM sleep stage (N2) the four elements, including the very low frequency component (VLF) of heart rate variability, the ratio of the low frequency component (LF) to the high frequency component (HF) (LF/HF ratio), the mean heartbeat interval (RRI), and the standard deviation of each heartbeat interval (RRI), indicate can be determined by majority vote to determine a sleep stage.

The learning unit 102c is a machine learning means that takes the heart rate variability parameters as inputs and that learns from training data for which sleep stages are known, which is stored in the storage unit 106. The learning unit 102c may use a known learning technique, such as a neural network (NN), a support vector machine (SVM), a random forest (RF), a recurrent neural network (RNN), or a hidden Markov model (HMM), as an algorithm for the learning. A specific example of the learning technique will be described below.

In Fig. 1, furthermore, the communication control interface unit 104 is a device that controls communication between the sleep determination apparatus 100 and a network 300 (or a communication device such as a router). That is, the communication control interface unit 104 has a function of communicating data to any other external device 200 or a station via a communication line (either wired or wireless). The network 300 has a function of interconnecting the sleep determination apparatus 100 and the external device 200, and examples of the network 300 include the Internet.

The sleep determination apparatus 100 may be communicably connected to the external device 200, which provides various databases such as for training data, heart rate signals, and heart rate variability parameters, an external program such as a sleep determination program according to the present invention, and so on, via the network 300. Alternatively, the sleep determination apparatus 100 may be communicably connected to the network 300 via a communication device, such as a router, and a wired or wireless communication line, such as a dedicated line.

In Fig. 1, furthermore, the external device 200, which is interconnected to the sleep determination apparatus 100 via the network 300, may have a function of providing training data or data such as for heart rate signals and heart rate variability parameters, an external database related to a determination technique or a sleep determination program, and a website for executing an external program such as a program or the like. The external device 200 may be configured as a WEB server, an ASP server, or the like, and may have a hardware configuration that is constituted by an information processing apparatus such as a commercially available workstation or personal computer, and it accessory devices. The functions of the external device 200 are implemented by elements of the hardware configuration of the external device 200, such as a CPU, a disk device, a memory device, an input device, an output device, and a communication control device, and by a program or the like that controls these devices.

When determining a sleep stage, the sleep determination apparatus 100 can use the determination technique described above solely or use a plurality of determination techniques in combination.

The description of the configuration of the sleep determination apparatus 100 according to this embodiment is complete.


[Processing in Sleep Determination Method]

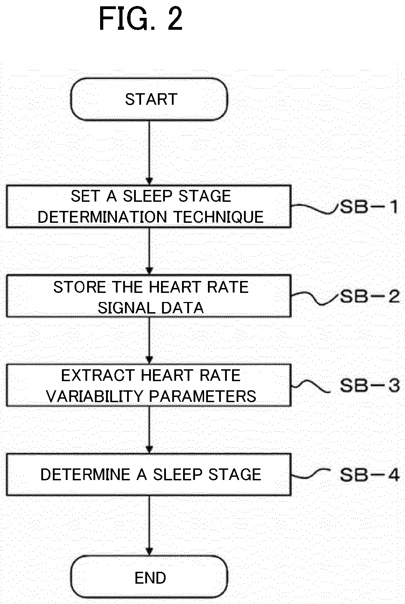

Next, an example of processing performed by the sleep determination apparatus 100 according to this embodiment having the configuration described above will be described in detail hereinafter with reference to Figs. 2 to 8. Fig. 2 is a flowchart illustrating an example of a sleep determination process performed by the sleep determination apparatus 100 according to this embodiment. The following process is an example technique for sleep determination using a machine learning algorithm.

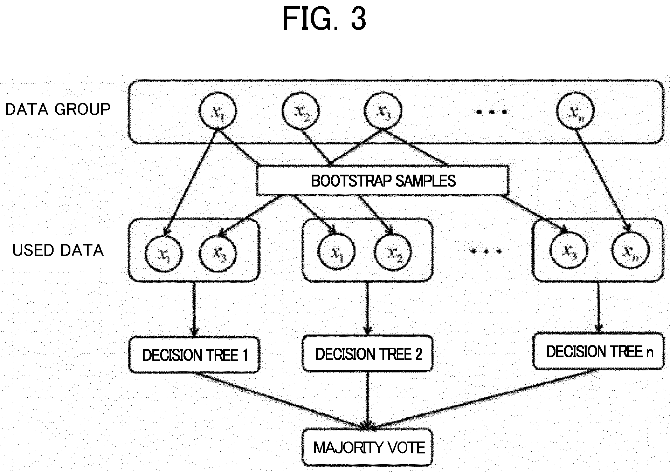

As illustrated in Fig. 2, first, the control unit 102 of the sleep determination apparatus 100 sets a sleep stage determination technique (step SB-1). For example, the sleep stage determination unit 102b of the control unit 102 may set the mathematical formula or algorithm stored in the determination technique file 106b as a determination technique. As an example, the sleep stage determination unit 102b may set, as a determination technique, learning result data that is obtained in advance by the learning unit 102c learning, from training data for which sleep stages are known, using the heart rate variability parameters as inputs. Fig. 3 is a diagram schematically illustrating, as a learning algorithm, an ensemble learning technique using decision trees in a random forest (RF).

As illustrated in Fig. 3, a random forest is an ensemble learning algorithm that uses decision trees as weak learners. In the random forest, (1) first, sub-samples to be used (bootstrap samples) are generated via random sampling from observation data to be learned (in this embodiment, a dataset in which explanatory variables such as the HF value, the LF value, the HF/LF ratio, and the mean or standard deviation of each RRI are associated with sleep stage classes). Then, (2) the sub-samples are used as training data to generate n decision trees. Then, (3) m explanatory variables among the explanatory variables in the training data are randomly selected to determine a split function at each node using one of the selected explanatory variables that can most exactly classify the training data and a threshold value therefor. Among the outputs of the decision trees with a predetermined number of nodes, which have been generated in the way described above, the final output is determined by majority vote on the output class and is evaluated. The feature value importance (contribution) of each explanatory variable can be calculated using the following equation.

[Math. 2]

$$I_m = \frac{1}{N}\sum_{i=1}^{N}\left(A_i - (B_m)_i\right)$$

N: Number of explanatory variables (recommended value: (number of feature dimensions)$^{1/2}$)
$A_i$: Percentage of correct answers obtained by correct classification using OOB data for the i-th decision tree
$(B_m)_i$: Percentage of correct answers obtained by inputting to the decision tree i data obtained by random permutation of the data of the feature value m in the OOB data
$I_m$: Importance of the feature value m

Here, a feature matrix is represented by the following expression.

[Math. 3]

$$\begin{pmatrix} HR_1 & LF_1 & HF_1 & LF_1/HF_1 & WK \\ HR_2 & LF_2 & HF_2 & LF_2/HF_2 & REM \\ \vdots & \vdots & \vdots & \vdots & \vdots \\ HR_n & LF_n & HF_n & LF_n/HF_n & WK \end{pmatrix}$$

where HR denotes the heart rate, LF denotes the total power value of the LF component, n denotes the number of observations, and HF denotes the total power value of the HF component.

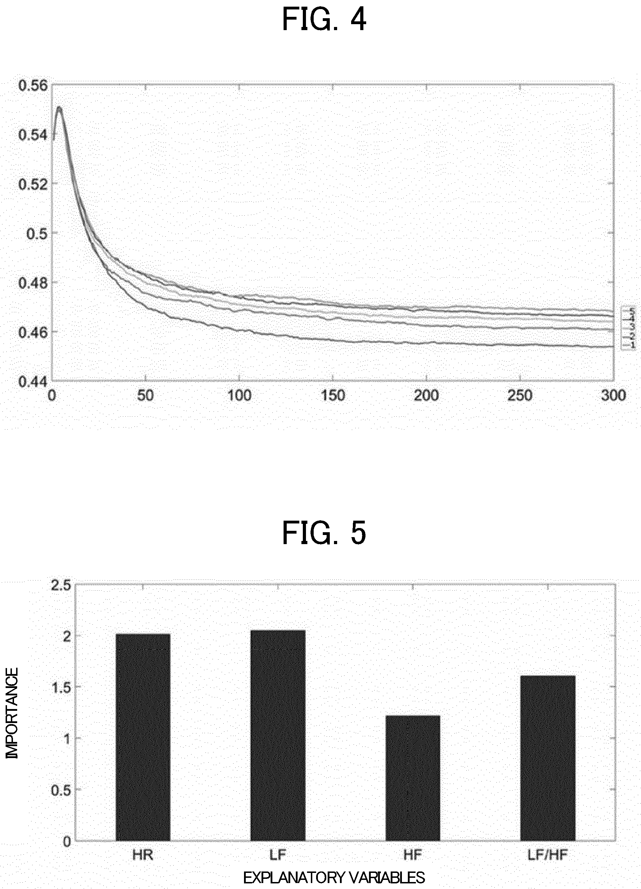

Fig. 4 is a graph chart illustrating a relationship of changes in prediction error with respect to the number of nodes and the number of decision trees in the case of permutation of explanatory variables. As illustrated in Fig. 4, the tendency is confirmed that as the number of nodes decreases and the number of decision trees increases, prediction error decreases. Fig. 5 is a graph chart illustrating the importance of the explanatory variables determined using the equation described above. As illustrated in Fig. 5, it is indicated that the importance increases in order of the HF and the LF/HF ratio, with the heart rate (HF) and the LF exhibiting the highest importance. The following table is a table showing the rate of identification (%), in one-vs-rest classification to discriminate between the first non-REM sleep stage (N1) and the other sleep stages, when the explanatory variable is subtracted from the classification set. It is confirmed that the first non-REM sleep (N1), which has been difficult to determine using techniques of the related art, is successfully determined as appropriate.

[Table 1]

| ONE-VS-REST CLASSIFICATION | |
|---|---|
| CLASSIFICATION SET | RATE OF IDENTIFICATION [%] |
| WK-VS-REST | 94.7 |
| REM-VS-REST | 80.7 |
| N1-VS-REST | 91.8 |
| N2-VS-REST | 63.1 |
| N3-VS-REST | 82.0 |

The following table is a chart illustrating the rate of identification (%) when four classes out of five stages including wake (WK), REM sleep (REM), the first non-REM sleep stage (N1), the second non-REM sleep stage (N2), and the third non-REM sleep stage (N3) are classified. It is confirmed that the random forest achieves relatively accurate classification.

[Table 2]

| FOUR-CLASS CLASSIFICATION | |
|---|---|
| CLASSIFICATION SET | RATE OF IDENTIFICATION [%] |
| OTHER THAN WK | 54.9 |
| OTHER THAN REM | 62.6 |
| OTHER THAN N1 | 60.3 |
| OTHER THAN N2 | 65.8 |
| OTHER THAN N3 | 66.2 |

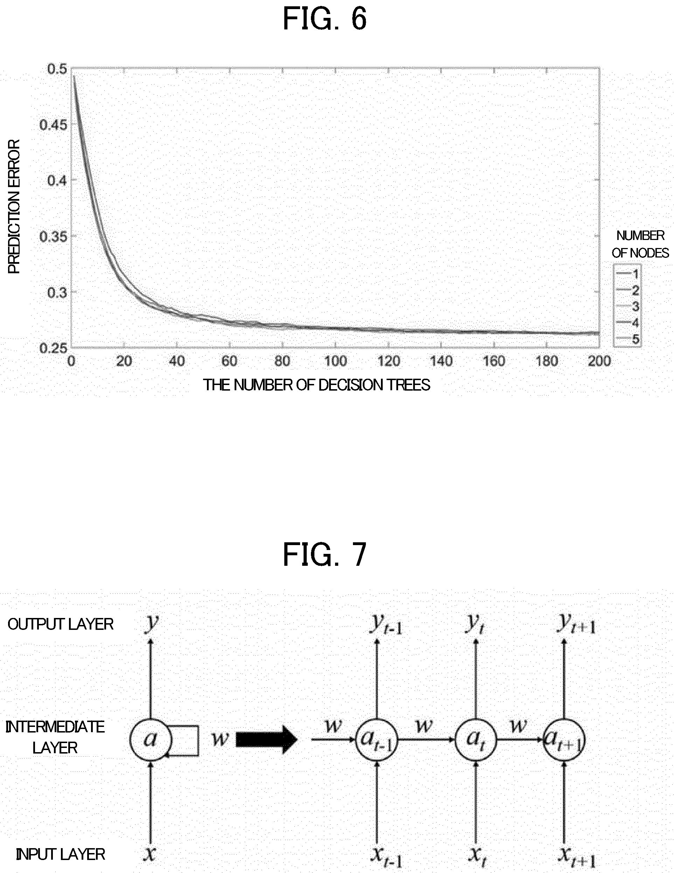

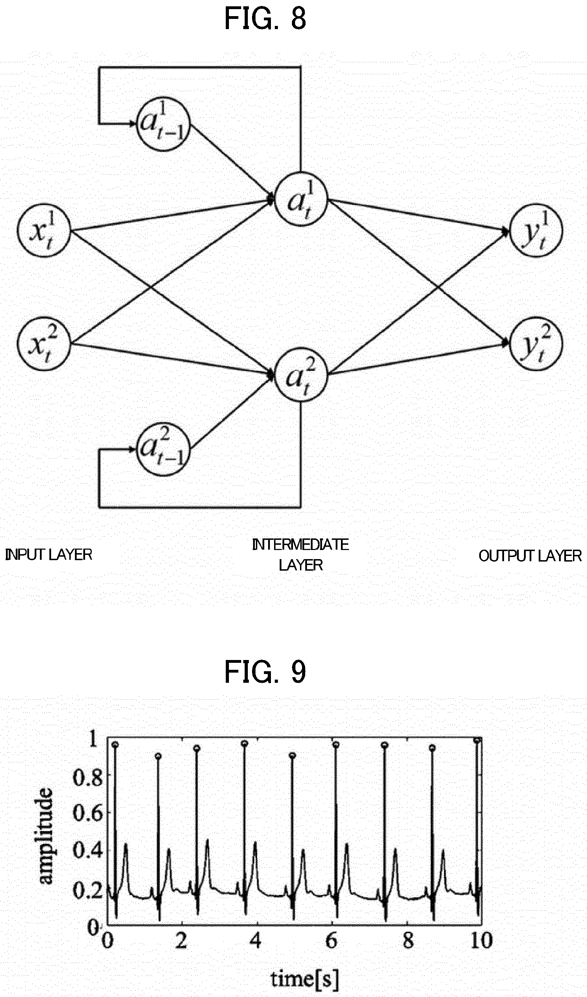

Fig. 6 is a diagram illustrating a relationship between the number of nodes and the prediction error in a random forest. As illustrated in Fig. 6, it is confirmed that as the number of decision trees increases, the prediction error decreases, without largely depending on the number of nodes. Instead of a random forest, as illustrated in Fig. 7 or 8, a recurrent neural network (RNN) may be used as a learning technique such that recurrence occurs at nodes. In typical neural networks, pieces of input data are handled as being independent from each other. In contrast, recurrent neural networks (RNNs), in which consecutive pieces of information are used, are generally used for audio recognition, machine translation, and so on. A known RNN technique can be applied to this embodiment. Figs. 7 and 8 are conceptual diagrams schematically illustrating a recurrent neural network (RNN). The following equation is a diagram illustrating a recurrence formula of propagation in the neural network illustrated in Fig. 7. Here, f denotes an activation function, and w denotes a weight.

[Math. 4]

$$a_t^j = f\left(\sum_i^N w^{ji} x_t^i + \sum_{j'}^N w^{jj'} a_{t-1}^j\right)$$

Alternatively, the learning unit 102c may derive learning results using a known learning technique such as a support vector machine (SVM) or a hidden Markov model (HMM), and set a determination technique.

Referring back to Fig. 2, subsequently, the control unit 102 of the sleep determination apparatus 100 stores the heart rate signal data input from the input unit 112 in the storage unit 106 (step SB-2). The storage unit 106 may store the heart rate signal data in advance, or the sleep determination apparatus 100 may receive the heart rate signal data stored in the external device 200 and store the heart rate signal data in the storage unit 106.

Then, the variable extraction unit 102a in the sleep determination apparatus 100 extracts heart rate variability parameters to be used for the determination technique set in step SB-1 from the heart rate variability data stored in the storage unit 106 (step SB-2). For example, the variable extraction unit 102a may extract the VLF, the LF, the HF, the mean RRI, the standard deviation of each RRI, the LF/HF ratio, and so on by using a known heart rate variability parameter extraction technique.

Then, the sleep stage determination unit 102b in the sleep determination apparatus 100 determines a sleep stage using, as inputs, the heart rate variability parameters extracted by the variable extraction unit 102a, in accordance with the determination technique set in step SB-1 (step SB-4). As an example useful for understanding the present invention, the sleep stage determination unit 102b may determine light sleep among the sleep stages using one-vs-rest classification. For example, the sleep stage determination unit 102b may discriminate between the light sleep stage including the first and second non-REM sleep stages (N1 & N2) and the other sleep stages. The following table is a diagram illustrating precisions (%) obtained as a result of one-vs-rest classification for N2 and the others.

[Table 3]

| N2 |
|---|
| 92.7 |
| 84.8 |
| 85.2 |
| 58.0 |
| 66.0 |
| 83.7 |
| 78.4 |
| 86.4 |
| 84.4 |
| 85.1 |
| 83.9 |
| 97.0 |
| 93.9 |

Generally, the sleep stage determination unit 102b is configured to perform multiclass classification. According to the invention, the sleep stage determination unit 102b is configured to perform five-class classification using the REM sleep stage (REM), the first non-REM sleep stage (N1), the second non-REM sleep stage (N2), the third non-REM sleep stage (N3), and the fourth non-REM sleep stage (N4). Alternatively, but not according to the invention, the sleep stage determination unit 102b may perform four-class classification using, as sleep stages, the REM sleep stage (REM), the light sleep stage including the first and second non-REM sleep stages (N1 & N2), the third non-REM sleep stage (N3), and the fourth non-REM sleep stage (N4). The processing described above is an example of a sleep determination process performed by the sleep determination apparatus 100 according to this embodiment.

The following table is a chart that compares precisions obtained as a result of four-class classification and five-class classification according to this embodiment ("this study" in the table) with precisions obtained using the techniques of the related art (NPLs [1], [2], and [3]).

[Table 4]

COMPARISON WITH RELATED ART

| REFFERENCES | BIOLOGICAL SIGNAL | NUMBER OF SUBJECTS | NUMBER OF CLASSES | PRECISION |
|---|---|---|---|---|
| THS STUDY | ELECTROCARDIOGRAM | 50 (MISSING 5) | 4 | 0.12 |
| | | | 5 | 0.66 |
| Isa et al.[1] | ELECTROCARDIOGRAM | 16 | 4 | 0.60 |
| Tanida et al.[2] | ELECTROCARDIOGRAM | 10 | 4 | 0.56 |
| Fonseca et al.[3] | ELECTROCARDIOGRAM ·RE-SPIRATORY FLOW | 48 | 4 | 0.69 |

As in the above chart, it is confirmed that, compared to four-class classification in the techniques of the related art, four-class classification according to this non-claimed embodiment achieves accurate classification with a precision of 72%. It is further confirmed that, in this embodiment, five-class classification, which has failed in the techniques of the related art, also achieves accurate classification with a precision of 66%. In this embodiment, accordingly, if the heart rate and heart rate variability can be accurately extracted, five-stage determination for sleep stages can be performed using only this information. In the techniques of the related art, only four-stage determination can be performed. In this embodiment, five-stage determination can be performed with a high precision of 0.72, compared to four-stage determination with a maximum precision of 0.69 in the technique of the related art using the heart rate and respiratory flow. In addition, since whether breathing is present can be determined, sleep apnea syndrome can be detected. The heart rate can be measured every day, without using a PSG device, which is useful for prediction of the onset of cardiac disease such as angina pectoris.

In other words, the methods of the related art provide four-class classification in which classification is performed such that N1 and N2 are grouped into a single class. If light sleep is identified through classification with separation of N1 (light sleep) and N2, the rate of sleep disorders is determined, leading to the discovery of a clinically significant category. In addition, N3 tends to decrease with age (reference: Hideto HIRASAWA and Yoshikata ATSUMI, "Polysomnographic Changes in Normal Human Aging", Japanese Journal of Geriatrics, Vol. 34, No. 6 (June 1997)), and N1 tends to increase with age. A new relationship between age and N1 can be found. It is said that risk for hypertension or diabetes mellitus with an increase in N1 increases, and such diseases may be prevented (see Md Basunia, et al., "Relationship of symptoms with sleep-stage abnormalities in obstructive sleep apnea-hypopnea syndrome", J Community Hosp Intern Med Perspect. 2016; 6(4):32170, and Denis Martinez et al., "Dimensions of sleepiness and their correlations with sleep-disordered breathing in mild sleep apnea", J. bras. pneumol. vol. 35 no. 6, June 2009). The method for classifying N1 and N2 previously requires attachment of a PSG device and testing in the hospital. In contrast, a sleep stage can be determined only using the heart rate, and the need for such capability is expected to grow.

In the related art, furthermore, there has been a technique for sleep stage determination using body movement or the like in which REM/non-REM sleep is determined only using information indicating whether there is a movement. In this embodiment, in contrast, the heart rate is accurately measured, and a correlation between determination values for sleep-stage determination and heart rates, which is obtained from PSG, is used to determine a sleep stage through learning. As a result, it is possible to identify five stages, including REM, non-REM, non-REM 1 to 3, and wake, with a precision of 72% being achieved currently because of an extremely small number of subjects having N1 data. Adaptive learning of N1 data expects further improvements in accuracy. That is, each time data of an individual is collected, the data is adaptively learned online, resulting in an expectation that the recognition rate is increasingly improved.

In this embodiment, furthermore, to classify these stages, heart rate feature values are determined and selected as appropriate. Training data with correct answers obtained through sleep-stage determination in a PSG test using only heart rates that are actually measured during sleep with attachment of a PSG device is used. Various kinds of noise are included, examples of which include noise caused by the extrinsic factor such as body movement or detachment of sensors during sleep, and noise caused on the device side when heart rate variability is obtained. To obtain the true heart rates from heart rate data containing these kinds of noise, it has taken 15 years or more for the present inventors to develop a technique for separating a signal obtained from a biological signal from noise. As a result of intensive studies, the present inventors have created this embodiment.

According to this embodiment, the use of a genetic algorithm among these techniques to perform five-class classification leads to the discovery of the importance of each feature value. It has been discovered that to perform five-class classification, the mean RRI, the standard deviation of each RRI, the VLF (0.003-0.04 Hz), the LF (0.04-0.15 Hz), the HF (0.15-0.4 Hz), and the LF/HF ratio are important. This embodiment further makes it possible to simultaneously classify these feature values for all of NN (Neural Network), SVM (Support Vector Machine), RF (Random Forest), RNN (Recurrent Neural Network), and HMM (Hidden Markov Model) classifiers. These learning algorithms can be used differently for different purposes. For example, RF is optimum to increase the rate of classification only for N2, and makes it possible to achieve high recognition, as illustrated in the chart described above, [Table 3]. In contrast, RNN is the most suitable for uniform recognition. According to this embodiment, as illustrated in the chart described above, [Table 4], the result of 66% or more can be obtained.

As described above, once the heart rate is obtained, this information is tagged with sleep information obtained from the PSG device, thereby enabling tagging such that the heart rate feature in sleep stage A is A1, the heart rate feature in sleep stage B is B1, and so on. Accordingly, it is possible to determine a sleep stage only using a heart rate feature. In addition, these results are learned using deep learning, and can thus be applied to various people.

[Example]

Next, an example according to the embodiment described above was conducted. The example may be similar to part of the embodiment described above.

The human heartbeat interval varies based on the action of sympathetic and parasympathetic nerves. The changes in heart rate variability are typically compared using the LF/HF ratio. With a human's transition from the wake state to the sleep state, the LF/HF ratio decreases. The recovery of the body during sleep decreases the basal metabolic rate and activates the parasympathetic nerves. As a result, the heart rate and its variability are considered to decrease, with LF/HF decreasing. The LF/HF ratio varies from one sleep stage to another. The LF/HF ratio in REM sleep is significantly larger than that in any other sleep stage. It is considered that during REM sleep, the basal metabolic rate increases, the sympathetic nerves are activated, and the LF/HF ratio increases. Additionally, it is said that there is a correlation between heart rate variability and EEG (electroencephalogram) in sleep.

Some researchers (Dumont et al.) showed that there was a cross-correlation between heart rate variability and EEG (electroencephalogram) during respiration by using SL (Synchronization Likehood) (see M. Dumont, F. Jurysta, J. Lanquart, P. Migeotte, P. Borne, and P. Linkowski, "Interdependency between heart rate variability and sleep EEG: linear/non-linear?", Clinical Neurophysiology, Vol. 115, No. 9, pp. 2031-2040, (2004)).

Other researchers (Yeh et al.) found that there was a fractal property between heart rate variability and EEG during sleep by using DFA (Detrended Fluctuation Analysis) (see J.R. Yeh, C.K. Peng, M.T. Lo, C.H. Yeh, S.C. Chen, C.Y. Wang, P.L. Lee, and J.H. Kang, "Investigating the interaction between heart rate variability and sleep EEG using nonlinear algorithms", Journal of Neuroscience Methods, Vol. 219, No. 2, pp. 233-239, (2013)). Since a sleep stage is determined mainly from EEG (electroencephalogram), any evidence that there is a correlation between heart rate variability and EEG index suggests the successful determination of a sleep stage using heart rate variability, which was examined in this example.

[Extraction of Feature Value and Classification of Sleep Stages]

First, an analysis procedure will be described for determining a sleep stage using ECG (electrocardiogram) . In the first stage of the analysis procedure, a bandpass filter from 5 to 15 Hz is applied to ECG to remove noise. Thereafter, the ECG is segmented into 30-second zones, and then, to enhance the R-wave peaks, the signal is squared to identify the R-wave. The R-wave is identified by shifting a window having a width of 0.5 seconds in increments of 0.25 seconds. After the R-wave is identified, the RRI is computed to generate RRI time-series data. Since the RRI is not a uniform interval, resampling is performed at a sampling frequency of 4 Hz by using spline interpolation. Then, a power spectral density (PSD) is computed from the RRI time-series data by using an AR model (autoregressive model) (see H. Akaike, "Fitting autoregressive models for prediction", Annals of the Institute of Statistical Mathematics, Vol. 21, No. 1, pp. 243-247, (1969)).

The power spectral density (PSD) is used to obtain the VLF, the LF, the HF, and the LF/HF ratio. The AR model provides frequency conversion, even for short time-series data, with higher frequency resolution than Fourier transform. In addition, the AR model is also suitable to obtain the deviation in peak frequency. In this example, the PSD is used for these reasons. The AR model is constructed using the Yule-Walker method, and the order of the AR model is selected on the basis of the following Akaike information criterion.

$$AIC = -2log\ f(x,\ \theta)\ +\ 2p$$

(where x denotes the observation variable, θ denotes the deviation in prediction error, f(x, θ) denotes the likelihood of the variable, and p denotes the number of variables.)

The above equation is minimized to determine an AR model.

The PSD (power spectral density) is computed by the following equation.

[Math. 5]

$$PSD(f) = \sigma^2 \Big/ \left| 1 - \sum_{j=1}^{M} a_j e^{-2\pi i j f} \right|^2$$

(where a denotes the AR (autoregressive) coefficient, and M denotes the order of the AR model: in this example, M was experimentally set to 2.)

After the RRI time-series data is subjected to frequency conversion, 0.003 to 0.04 Hz (VLF), 0.004 to 0.15 Hz (LF), 0.15 to 0.4 Hz (HF), and the LF/HF ratio are computed from a set of power spectral densities. Through the analysis procedure described above, the RRI mean, the standard deviation of each RRI, the VLF, the LF, the HF, and the LF/HF ratio are computed as feature variables.

[Obtaining of Data]

To obtain a determination criterion in this example, PSG was performed on 50 subjects and a sleep experiment was conducted. Unfortunately, the equipment was separated from five subjects in the middle, and data for the five subjects was missing and excluded. In this example, a sleep stage was estimated using only ECG (electrocardiogram). The estimated sleep stage was matched against correct answer data to compute a recognition rate. Current data that was correctly estimated was used as the determination criterion described above.

It is generally said that, in normal sleep, REM, N2, and N3 occupy 25%, 50%, and 20% of the total sleep time, respectively. In this example, as shown in the following table, REM occupied 21%, N2 occupied 60%, and N3 occupied 10%. In a PSG test, the occurrence ratio of N3, which is deep sleep, appears to be kept low.

[Table 5]

The number of data in each stage

| | |
|---|---|
| WK | 2942 |
| REM | 5944 |
| N1 | 2295 |
| N2 | 16790 |
| N3 | 3091 |

[Data Analysis]

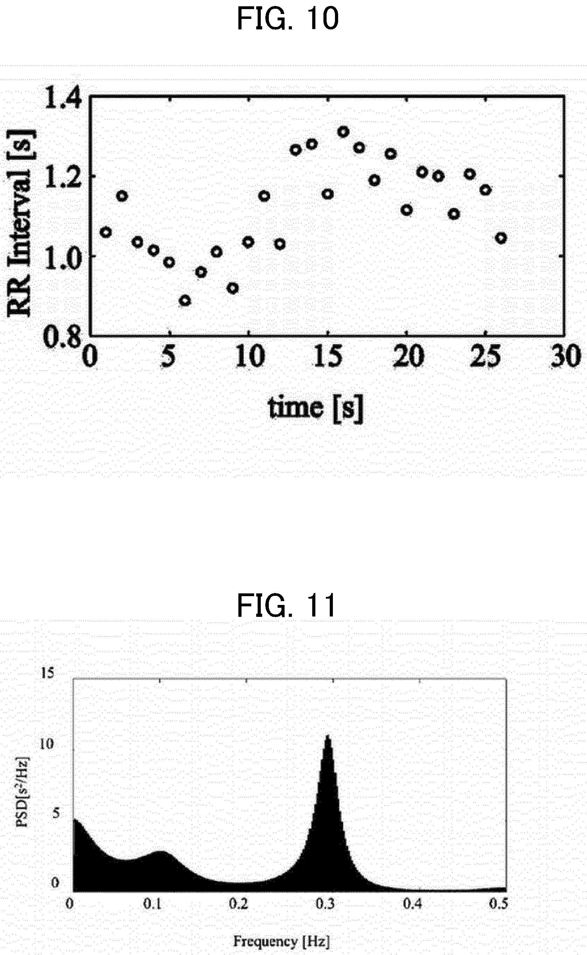

The R-wave and the RRI time-series data were identified from the ECG data (see Figs. 9 and 10), and the power spectral density of the RRI was determined (see Fig. 11). Fig. 9 is a diagram illustrating an example of the result of R-wave detection, Fig. 10 is a diagram illustrating an example of the RRI time-series data, and Fig. 11 is a diagram illustrating PSD data computed from the RRI.

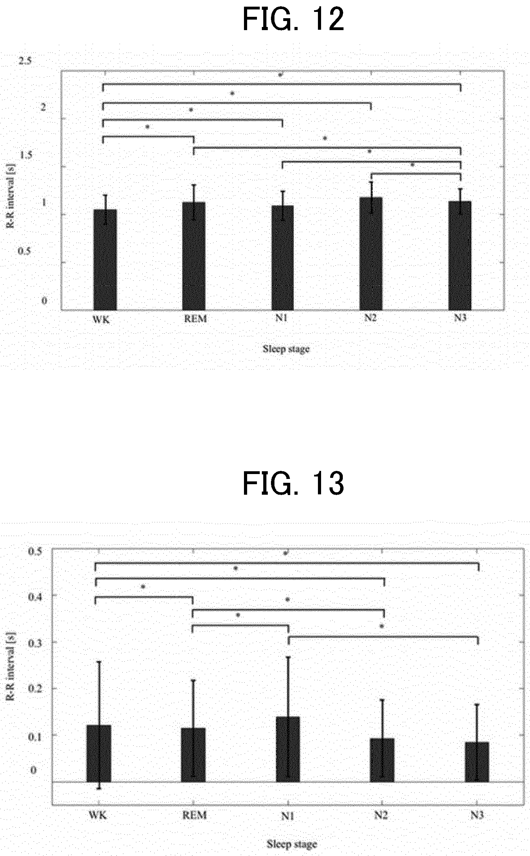

Subsequently, as illustrated in Figs. 12 and 13, a multiple comparison test was conducted, with the mean and significance level of feature values being set to 5% for all the subjects in each sleep stage. Fig. 12 is a diagram illustrating the RRI in each sleep stage, and Fig. 13 is a diagram illustrating the standard deviation in each sleep stage.

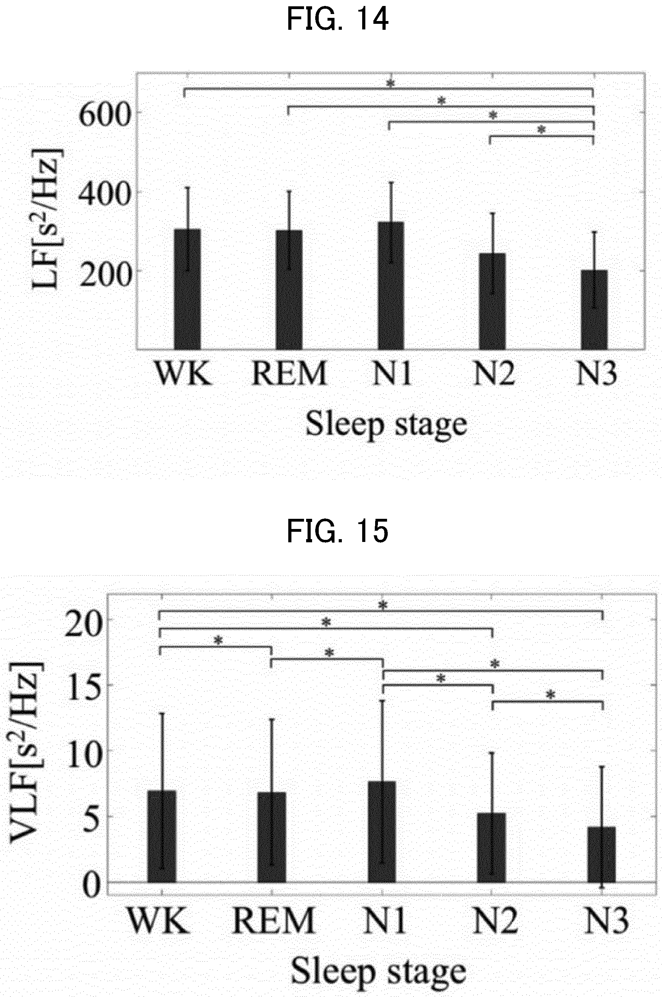

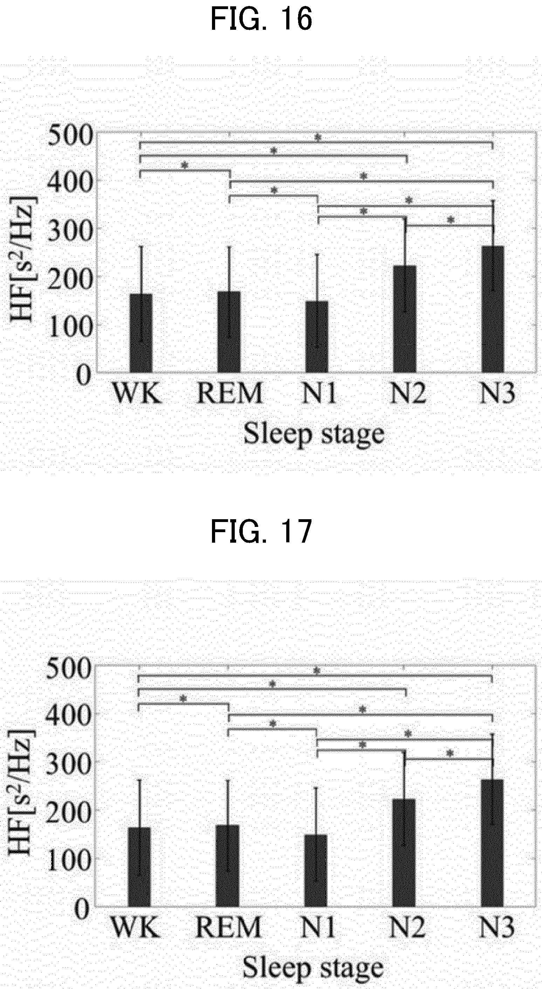

Fig. 12 indicates that WK and N3 were significantly different from REM, N1, and N2. On the other hand, no significant difference was found among REM, N1, and N2. The heart rate during sleep was lower than that during WK (wake). It is said that the heart rate decreases from WK to N3. Also in this example, a similar tendency was detected. However, the heart rate was lower in N2 than in N3. Additionally, the standard deviation of heart rate variability is said to be maximum in the REM stage and also in WK, but such a tendency was not observed in this example, as in Fig. 13. The heart rate variability in N1 was higher than that in WK. Fig. 14 is a diagram illustrating the LF in each sleep stage, Fig. 15 is a diagram illustrating the VLF in each sleep stage, Fig. 16 is a diagram illustrating the HF in each sleep stage, and Fig. 17 is a diagram illustrating the LF/HF ratio in each sleep stage.

Figs. 15 and 16 confirm that WK was significantly different from all the sleep stages. It is said that as sleep becomes deep, the LF decreases and the HF increases. A similar tendency was observed also in this example.

[Classifiers for Identifying Sleep Stages]

Subsequently, a sleep stage was determined using the computed feature values. In this example, RNN, HMM, neural network (NN), support vector machine (SVM), and random forest (RF) were used as classifiers for sleep-stage determination. Whereas RNN and HMM are methods that take into account time-series information of observed values, NN, SVM, and RF handle each observed value as an independent value. The HMM requires vector quantization of feature values, and clustering and vector quantization are carried out using the SOM (see T. Kohonen, "Self-organizing formation of topologically correct feature maps", Biological Cybernetics, Vol. 43, pp. 59-69, (1982)). Thereafter, learning was performed by the Baum-Welch algorithm, and Viterbi algorithm was used to obtain outputs (see A.J. Viterbi, "Error bounds for convolutional codes and an asymptotically optimum decoding algorithm", IEEE Transactions on Information Theory, Vol. 13, No. 2, pp. 260-269, (1967)). One of the subjects was used as test data to perform leave-one-out cross-validation (LOOCV), and the remaining subjects were used as training data to evaluate the classifiers. Finally, the recognition rate of each sleep stage was determined to evaluate the classifiers. The recognition rate is expressed in the ratio of the number of correctly classified classes to the total number of classes.

[Results and Discussion]

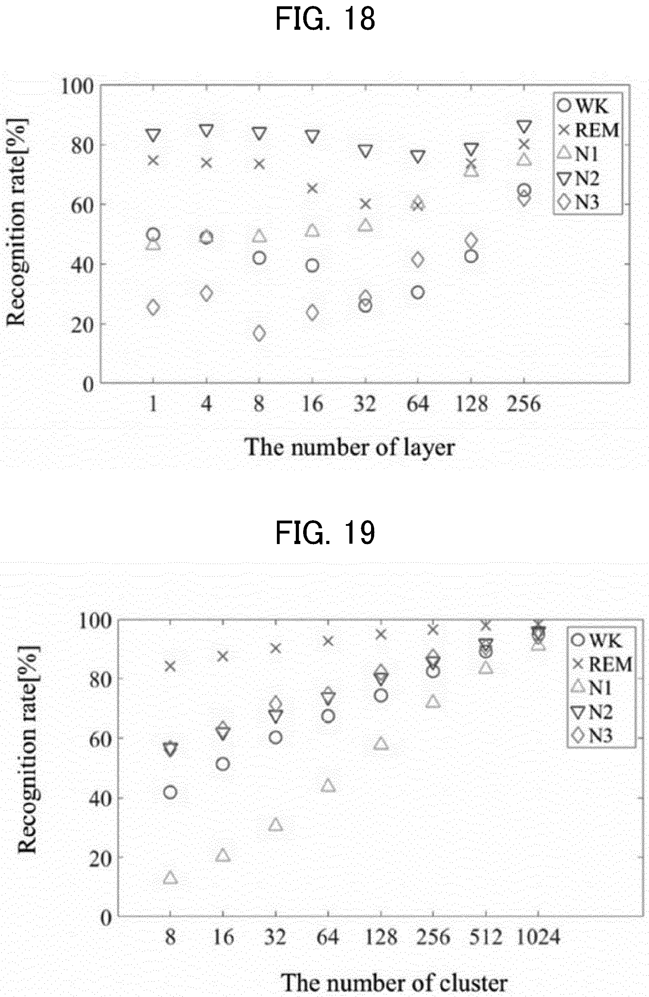

The recognition results of the sleep stages using heart rate variability are illustrated in Fig. 18. Fig. 18 is a diagram illustrating a relationship between the number of layers in the RNN and changes in recognition rate. The results indicate that as the number of layers increases, the recognition rate increases for all the sleep stages. This may also indicate that as the layer becomes deep, the expression capacity of the RNN increases.

Fig. 19 is a diagram illustrating a relationship between the number of clusters in the HMM and changes in recognition rate. It is found that when the number of clusters is 1024, a recognition rate of 80% or more is achieved for all the sleep stages.

Tables 6 and 7 below show results of recognition rates separately for learning and testing. RNN and HMM were applied to the test data.

[Table 6]

COMPARISON WITH RELATED ART

| REFFERENCES | BIOLOGICAL SIGNAL | NUMBER OF SUBJECTS | NUMBER OF CLASSES | PRECISION |
|---|---|---|---|---|
| THS STUDY | ELECTROCARDIOGRAM | 50 (MISSING 5) | 4 | 0.72 |
| | | | 5 | 0.66 |
| Isa et al.[1] | ELECTROCARDIOGRAM | 16 | 4 | 0.60 |
| Tanida et al.[2] | ELECTROCARDIOGRAM | 10 | 4 | 0.56 |
| Fonseca et al.[3] | ELECTROCARDIOGRAM ·RE-SPIRATORY FLOW | 48 | 4 | 0.69 |

Furthermore, NN, SVM, and RF were applied for comparison with the method according to this embodiment. As a result, outcomes shown in Table 7 below were obtained.

[Table 7]

| method | Bio-signal | subjects | class | Recognition rate |
|---|---|---|---|---|
| Proposed | ECG | 45 | 4 | 0.72 |
| | | | 5 | 0.66 |
| Isa *et al.* | ECG | 16 | 4 | 0.60 |

(continued)

| method | Bio-signal | subjects | class | Recognition rate |
|---|---|---|---|---|
| Tanida *et al.* | ECG | 22 | 4 | 0.56 |
| Fonseca *et al.* | ECG, RIP | 48 | 4 | 0.69 |
| Willemen *et al.* | ECG, RIP, ACT | 85 | 4 | 0.69 |

The outcomes indicate that the method according to this embodiment ("Proposed" in the table) achieved relatively higher recognition accuracy than using RNN as a classifier. Compared to various methods, a higher recognition rate was obtained than that in a method that does not take into account a time information method. Since sleep stages do not markedly change, the use of time-series information may improve the recognition rate. In NN and SVM, learning was performed to minimize classification errors in the entire data, with the result that other classes were erroneously classified into N2 for which the number of pieces of data is large. In this example, the number of pieces of N2 data was large, whereas the number of pieces of N1 data and the number of pieces of N3 data were small. The increase in the number of pieces of data leaded to an increase in recognition rate.

As a result of comparison between the method according to this embodiment and the methods of the related art, the outcomes shown in Table 7 were obtained (see NPLs 1 to 3 and T. Willemen, D.V. Deun, V. Verhaert, M. Vandekerckhove, V. Exadaktylos, J. Verbraecken, S.V. Huffel, B. Haex, and J.V. Sloten, "An evaluation of cardiorespiratory and movement features with respect to sleep-stage classification", IEEE Journal of Biomedical and Health Informatics, Vol. 18, No. 2, pp. 661-669, (2014)). RIP (respiratory inductance plethysmography) measures the respiratory flow rate, and ACT (actigraphy) measures the movement of the body, which is measured by accelerometers on the arms or ankles.

There is no reported criterion for five stages of sleep using ECG. Many determinations have been based on four stages of sleep, in which N1 and N2 are combined into a single stage. N1, which is light sleep, is important as a sleep stage. Some studies have reported that Apnea-hypopnea index (AHI) and N2 have a significant correlation and have a correlation with the maximum dimension of excessive daytime sleepiness EDS. Furthermore, the results of multivariate regression analysis have reported that AHI-N1 has a significant correlation with PSG factors (sleep efficiency, sleep urgency) and that AHI-N2 has a significant correlation with the chief complaint such as daytime sleepiness (drowsiness) or insomnia (see Denis Martinez, Magali Santos Lumertz, and Maria do Carmo Sfreddo Lenz, "Dimensions of sleepiness and their correlations with sleep-disordered breathing in mild sleep apnea", J Bras Pneumol. 2009;35(6):507-514). It is therefore considered that recognition of five sleep stages, in which the N1 and N2 sleep stages are separate, is important.

[Conclusions]

In this example, five sleep stages were recognized using only ECG. The methods of the related art are methods for four-stage recognition in which N1 and N2 are combined into a single stage. In contrast, the method according to this embodiment can identify five stages in which N1 and N2 are separate. According to this embodiment, as a result of identifying four stages in a similar way to that in the methods of the related art that use RIP and ACT data in addition to ECG, outcomes with higher recognition rate than those in the methods of the related art can be obtained. According to this embodiment, furthermore, it is possible to identify five stages in a similar way to that in the methods of the related art for identifying four stages. One of the state-of-the-art research findings indicates that the REM sleep stage (REM), the first non-REM sleep stage (N1), the second non-REM sleep stage (N2), the third non-REM sleep stage (N3), and the wake state can be identified with accuracies of 0.86, 0.93, 0.69, 0.90, and 0.93, respectively, as the recognition rates for the sleep stages. There has been no report for an identification method for five stages of sleep using ECG. Many methods of the related art merely provide identification of four stages in which N1 and N2 are regarded as a single stage. For sleep disorders or EDS, N1 and N2 need to be separated, and therefore the five-stage identification method according to this embodiment has high value in use. In this example, the recognition rate for N1 was low because the number of pieces of N1 data was not so large, whereas the recognition rate for N2 was as high as 90% or more. It is considered that the use of data including a large number of pieces of N1 data further improves the recognition rate for N1.

The description of this embodiment is complete.

[Other Embodiments]

While an embodiment of the present invention has been described, the present invention may be carried out in various different embodiments, other than the embodiment described above, without departing from the scope of the invention defined in the claims.

For example, the sleep determination apparatus 100 has been described as performing processing in a standalone mode. However, the sleep determination apparatus 100 may be configured to perform processing in response to a request from a client terminal (such as the external device 200) and to return the result of the processing to the client terminal.

Furthermore, among the processes described in the embodiment, all or some of the processes described as being automatically performed may be performed manually, or all or some of the processes described as being manually performed may be performed automatically using a known method.

In addition, the processing procedures, the control procedures, the specific names, the information including registered data and parameters, such as search conditions, for each processing operation, the example screens, and the database configurations, which are provided in the literatures described above or illustrated in the drawings, may be changed, as desired, unless otherwise stated.

The illustrated components of the sleep determination apparatus 100 are functionally conceptual, and need not be physically configured as illustrated in the drawings.

For example, the processes and functions of the devices in the sleep determination apparatus 100, in particular, the processes and functions performed by the control unit 102, may be implemented in part or in whole using a CPU (Central Processing Unit) and a program interpreted and executed by the CPU, or may be implemented in part or in whole as wired-logic-based hardware. The program is recorded on a non-transitory computer-readable recording medium described below, which includes instructions programmed to cause a computer to perform a method according to the present invention. The program is mechanically loaded into the sleep determination apparatus 100, as necessary. That is, the storage unit 106, such as a ROM or an HDD (Hard Disk Drive), has recorded thereon a computer program that cooperates with an OS (Operating System) to provide instructions to the CPU to perform various processing operations. The computer program is executed by being loaded into the RAM. The computer program cooperates with the CPU to form a control unit.

The computer program may be stored in an application program server connected to the sleep determination apparatus 100 via any desired network 300, and may be downloaded in full or in part, as necessary.

A program according to the present invention may be stored in a computer-readable recording medium, or may be configured as a program product. The "recording medium", as used here, includes any "portable physical medium" such as a memory card, a USB memory, an SD card, a flexible disk, a magneto-optical disk, a ROM, an EPROM, an EEPROM, a CD-ROM, an MO, a DVD, and a Blu-ray (registered trademark) Disc.

The "program" refers to a data processing method written in any desired language or writing method, and may be in the format of source code or binary code or in any other format. The "program" is not necessarily limited to a program configured as a single unit, and includes a program configured in a distributed manner as a plurality of modules or libraries, or includes a program that cooperates with a separate program, such as an OS (Operating System), to achieve its function. A specific configuration of each device provided in the embodiment for reading a recording medium, a reading procedure, or an installation procedure or the like after reading may be implemented using a known configuration or procedure. The program may be configured as a program product recorded on a non-transitory computer-readable recording medium, and may constitute the present invention.

Various databases and the like (such as the heart rate variable file 106a and the determination technique file 106b) stored in the storage unit 106 are storage means such as a memory device such as a RAM or a ROM, a fixed disk device such as a hard disk, a flexible disk, and an optical disk, and store various programs, tables, databases, web page files, and so on to be used to perform various processing operations or provide a website.

The sleep determination apparatus 100 or the external device 200 may be configured as a known information processing apparatus such as a personal computer or a workstation, or may be configured such that any peripheral device is connected to the information processing apparatus. Alternatively, the sleep determination apparatus 100 or the external device 200 may be implemented by loading, to the information processing apparatus, software (including a program, data, and so on) for implementing the method according to the present invention.

Moreover, the devices may be distributed or integrated in any form other than that illustrated in the drawings. All or some of the devices may be configured to be distributed or integrated functionally or physically in any unit in accordance with various loads or the like or in accordance with the functional load. That is, an embodiment may be selectively implemented.

Industrial Applicability

As described above in detail, according to the present invention, it is possible to provide a sleep determination apparatus, a sleep determination method, and a sleep determination program that provide an accurate and detailed determination of a sleep stage.

EXPLANATION OF REFERENCE NUMERALS

100 sleep Determination Apparatus
102 control unit
102a variable extraction unit
102b sleep stage determination unit
102c learning unit
104 communication control interface unit
106 storage unit
106a heart rate variable file
106b determination technique file
108 input/output control interface unit
112 input unit
114 output unit
200 external device
300 network

**Claims**

1. A sleep determination apparatus (100) comprising:

   a heart rate data obtaining means (102a) for obtaining, based on data regarding a heart rate of a body of a user, a very low frequency component (VLF), a ratio of a low frequency component (LF) to a high frequency component (HF), a mean heartbeat interval (RRI), and a standard deviation of each heartbeat interval (RRI) of the heart rate as heart rate variability parameters indicating a heart rate state; and
   a sleep state determining means (102b) for determining which of a plurality of sleep stages the user is in;
   **characterized in that**
   the sleep state determining means (102b) is configured to determine the sleep stage in such a manner as to distinguish, as the sleep stages, five stages including a REM sleep stage (REM), and, in order of lightest sleep stage, a first non-REM sleep stage N1, a second non-REM sleep stage N2, a third non-REM sleep stage N3, and a fourth non-REM sleep stage N4, wherein
   light sleep is classified into the first non-REM sleep stage N1 and the second non-REM sleep stage N2; and
   the sleep state determining means (102b) is configured to determine which of the plurality of sleep stages the user is in from features indicated by a plurality of the heart rate variability parameters using only the heart rate variability parameters.

2. The sleep determination apparatus (100) according to claim 1,
   wherein the sleep state determining means (102b) is configured to determine which of the first non-REM sleep stage N1 or the second non-REM sleep stage N2 the heart rate variability parameters of the user indicate, in accordance with a first determination condition that is set based on a correlation between the heart rate variability parameters and the first non-REM sleep stage N1 and a second determination condition that is set based on a correlation between the heart rate variability parameters and the second non-REM sleep stage N2.

3. The sleep determination apparatus (100) according to claim 1,
   wherein the sleep state determining means (102b) is configured to determine a sleep stage from the heart rate variability parameters, the determination being based on learning result data that is obtained by machine learning, from training data for which the sleep stages are known, using the heart rate variability parameters as inputs.

4. The sleep determination apparatus (100) according to claim 3,
   wherein an algorithm for the machine learning is a neural network, a support vector machine, a random forest, a recurrent neural network, or a hidden Markov model.

5. The sleep determination apparatus (100) according to claim 1,
   wherein the sleep state determining means (102b) is configured to determine which of the sleep stages the user is in, the determination being based on a function indicating a probability of being in each of the plurality of sleep stages, the function having the heart rate variability parameters as variables, the plurality of sleep stages including the first non-REM sleep stage N1 and the second non-REM sleep stage N2.

6. A sleep determination method executed by a computer comprising:

a heart rate data obtaining step (SB-3) of obtaining, based on data regarding a heart rate of a body of a user, a very low frequency component (VLF), a ratio of a low frequency component (LF) to a high frequency component (HF), a mean heartbeat interval (RRI), and a standard deviation of each heartbeat interval (RRI) of the heart rate as heart rate variability parameters indicating a heart rate state; and
a sleep state determining step (SB-4) of determining which of a plurality of sleep stages the user is in;
**characterized in that**
in the sleep state determining step (SB-4), the sleep stage is determined in such a manner as to distinguish, as the sleep stages, five stages including a REM sleep stage (REM), and, in order of lightest sleep stage, a first non-REM sleep stage N1, a second non-REM sleep stage N2, a third non-REM sleep stage N3, and a fourth non-REM sleep stage N4, wherein
light sleep is classified into the first non-REM sleep stage N1 and the second non-REM sleep stage N2;
in the sleep state determining step (SB-4), it is determined which of the plurality of sleep stages the user is in from features indicated by a plurality of the heart rate variability parameters using only the heart rate variability parameters.

7. A sleep determination program for causing a computer to realize:

a heart rate data obtaining function of obtaining, based on data regarding a heart rate of a body of a user, a very low frequency component (VLF), a ratio of a low frequency component (LF) to a high frequency component (HF), a mean heartbeat interval (RRI), and a standard deviation of each heartbeat interval (RRI) of the heart rate as heart rate variability parameters indicating a heart rate state; and
a sleep state determining function of determining which of a plurality of sleep stages the user is in;
**characterized in that**
the sleep state determining function determines the sleep stage in such a manner as to distinguish, as the sleep stages, five stages including a REM sleep stage (REM), and, in order of lightest sleep stage, a first non-REM sleep stage N1, a second non-REM sleep stage N2, a third non-REM sleep stage N3, and a fourth non-REM sleep stage N4,
wherein
light sleep is classified into the first non-REM sleep stage N1 and the second non-REM sleep stage N2;
the sleep state determining function determines which of the plurality of sleep stages the user is in from features indicated by a plurality of the heart rate variability parameters using only the heart rate variability parameters.

**Patentansprüche**

1. Eine Schlafbestimmungsvorrichtung (100), die aufweist:

eine Erfassungseinrichtung (102a) für Herzfrequenzdaten, um auf der Basis von Daten bezüglich einer Herzfrequenz eines Körpers eines Benutzers eine sehr niederfrequente Komponente (VLF), ein Verhältnis einer niederfrequenten Komponente (LF) zu einer hochfrequenten Komponente (HF), ein mittleres Herzschlagintervall (RRI) und eine Standardabweichung jedes Herzschlagintervalls (RRI) der Herzfrequenz als Herzfrequenzvariabilitätsparameter zu erhalten, die einen Herzfrequenzzustand anzeigen; und
eine Bestimmungsvorrichtung für den Schlafzustand (102b) zum Bestimmen, in welchem von einer Vielzahl von Schlafstadien sich der Benutzer befindet;
**dadurch gekennzeichnet, dass**
die Bestimmungsvorrichtung für den Schlafzustand (102b) so konfiguriert ist, dass sie den Schlafzustand so bestimmt, dass sie als Schlafzustände fünf Zustände unterscheidet, einschließlich eines REM-Schlafzustands (REM) und, in der Reihenfolge des leichtesten Schlafzustands, eines ersten Nicht-REM-Schlafzustands N1, eines zweiten Nicht-REM-Schlafzustands N2, eines dritten Nicht-REM-Schlafzustands N3 und eines vierten Nicht-REM-Schlafzustands N4, wobei
leichter Schlaf in das erste Nicht-REM-Schlafstadium N1 und das zweite Nicht-REM-Schlafstadium N2 klassifiziert wird; und
die Bestimmungsvorrichtung für den Schlafzustand (102b) so konfiguriert ist, dass sie anhand von Merkmalen, die durch eine Vielzahl der Herzfrequenzvariabilitätsparameter angezeigt werden, bestimmt, in welchem der Vielzahl von Schlafstadien sich der Benutzer befindet, wobei sie nur die Herzfrequenzvariabilitätsparameter verwendet.

2. Die Schlafbestimmungsvorrichtung (100) nach Anspruch 1,
wobei die Bestimmungsvorrichtung für den Schlafzustand (102b) so konfiguriert ist, dass sie gemäß einer ersten Bestimmungsbedingung, die auf der Basis einer Korrelation zwischen den Herzfrequenzvariabilitätsparametern und dem ersten Nicht-REM-Schlafstadium N1 eingestellt ist, und einer zweiten Bestimmungsbedingung, die auf der Basis einer Korrelation zwischen den Herzfrequenzvariabilitätsparametern und dem zweiten Nicht-REM-Schlafstadium N2 eingestellt ist, bestimmt, welches des ersten Nicht-REM-Schlafstadiums N1 oder des zweiten Nicht-REM-Schlafstadiums N2 die Herzfrequenzvariabilitätsparameter des Benutzers anzeigen.

3. Die Schlafbestimmungsvorrichtung (100) nach Anspruch 1,
wobei die Bestimmungsvorrichtung für den Schlafzustand (102b) so konfiguriert ist, dass sie ein Schlafstadium aus den Herzfrequenzvariabilitätsparametern bestimmt, wobei die Bestimmung auf Lernergebnisdaten basiert, die durch maschinelles Lernen aus Trainingsdaten erhalten werden, für die die Schlafstadien bekannt sind, wobei die Herzfrequenzvariabilitätsparameter als Eingaben verwendet werden.

4. Die Schlafbestimmungsvorrichtung (100) nach Anspruch 3,
wobei ein Algorithmus für das maschinelle Lernen ein neuronales Netzwerk, eine Support-Vektor-Maschine, ein Random Forest, ein rekurrentes neuronales Netzwerk oder ein Hidden-Markov-Modell ist.

5. Die Schlafbestimmungsvorrichtung (100) nach Anspruch 1,
wobei die Bestimmungsvorrichtung für den Schlafzustand (102b) so konfiguriert ist, dass sie bestimmt, in welchem der Schlafzustände sich der Benutzer befindet, wobei die Bestimmung auf einer Funktion basiert, die eine Wahrscheinlichkeit angibt, sich in jedem der Vielzahl von Schlafzuständen zu befinden, wobei die Funktion die Parameter der Herzfrequenzvariabilität als Variablen aufweist, wobei die Vielzahl von Schlafzuständen das erste Nicht-REM-Schlafstadium N1 und das zweite Nicht-REM-Schlafstadium N2 umfasst.

6. Ein Verfahren zur Bestimmung des Schlafes, das von einem Computer ausgeführt wird und Folgendes aufweist:

einen Schritt (SB-3) zur Gewinnung von Herzfrequenzdaten, um auf der Basis von Daten bezüglich einer Herzfrequenz eines Körpers eines Benutzers eine sehr niederfrequente Komponente (VLF), ein Verhältnis einer niederfrequenten Komponente (LF) zu einer hochfrequenten Komponente (HF), ein mittleres Herzschlagintervall (RRI) und eine Standardabweichung jedes Herzschlagintervalls (RRI) der Herzfrequenz als Herzfrequenzvariabilitätsparameter zu gewinnen, die einen Herzfrequenzzustand anzeigen; und
einen Schlafzustands-Bestimmungsschritt (SB-4), in dem bestimmt wird, in welchem von einer Vielzahl von Schlafstadien sich der Benutzer befindet;
**dadurch gekennzeichnet, dass**
in dem Schlafzustands-Bestimmungsschritt (SB-4) das Schlafstadium auf eine solche Weise bestimmt wird, dass als die Schlafstadien fünf Stadien unterschieden werden, einschließlich eines REM-Schlafstadiums (REM) und, in der Reihenfolge des leichtesten Schlafstadiums, eines ersten Nicht-REM-Schlafstadiums N1, eines zweiten Nicht-REM-Schlafstadiums N2, eines dritten Nicht-REM-Schlafstadiums N3 und eines vierten Nicht-REM-Schlafstadiums N4, wobei
leichter Schlaf in das erste Nicht-REM-Schlafstadium N1 und das zweite Nicht-REM-Schlafstadium N2 klassifiziert wird;
in dem Schlafzustands-Bestimmungsschritt (SB-4) wird anhand von Merkmalen, die durch eine Vielzahl der Herzfrequenz-Variabilitätsparameter angezeigt werden, bestimmt, in welchem der Vielzahl von Schlafstadien sich der Benutzer befindet, wobei nur die Herzfrequenz-Variabilitätsparameter verwendet werden.

7. Ein Schlafbestimmungsprogramm, um einen Computer zu veranlassen, folgendes zu realisieren:

eine Funktion zur Gewinnung von Herzfrequenzdaten, um auf der Basis von Daten bezüglich einer Herzfrequenz eines Körpers eines Benutzers eine sehr niederfrequente Komponente (VLF), ein Verhältnis einer niederfrequenten Komponente (LF) zu einer hochfrequenten Komponente (HF), ein mittleres Herzschlagintervall (RRI) und eine Standardabweichung jedes Herzschlagintervalls (RRI) der Herzfrequenz als Herzfrequenzvariabilitätsparameter zu gewinnen, die einen Herzfrequenzzustand anzeigen; und
eine Bestimmungsfunktion für den Schlafzustand, die bestimmt, in welchem von einer Vielzahl von Schlafstadien sich der Benutzer befindet;

**dadurch gekennzeichnet, dass**

die Bestimmungsfunktion für den Schlafzustand das Schlafstadium auf eine solche Weise bestimmt, dass sie als die Schlafstadien fünf Stadien unterscheidet, einschließlich eines REM-Schlafstadiums (REM) und, in der Reihenfolge des leichtesten Schlafstadiums, eines ersten Nicht-REM-Schlafstadiums N1, eines zweiten Nicht-REM-Schlafstadiums N2, eines dritten Nicht-REM-Schlafstadiums N3 und eines vierten Nicht-REM-Schlafstadiums N4, wobei

leichter Schlaf in das erste Nicht-REM-Schlafstadium N1 und das zweite Nicht-REM-Schlafstadium N2 klassifiziert wird;

die Bestimmungsfunktion für den Schlafzustand bestimmt, in welchem der Vielzahl von Schlafstadien sich der Benutzer befindet, anhand von Merkmalen, die durch eine Vielzahl von Parametern der Herzfrequenzvariabilität angezeigt werden, wobei nur die Parameter der Herzfrequenzvariabilität verwendet werden.

**Revendications**

1. Appareil de détermination de l'état de sommeil (100) comprenant :

   un moyen d'obtention de données de fréquence cardiaque (102a) pour obtenir, sur la base de données concernant une fréquence cardiaque du corps d'un utilisateur, une composante de très basse fréquence (VLF), un rapport d'une composante de basse fréquence (LF) à une composante de haute fréquence (HF), un intervalle de battement cardiaque (RRI) moyen, et un écart type de chaque intervalle de battement cardiaque (RRI) de la fréquence cardiaque en tant que paramètres de variabilité de la fréquence cardiaque indiquant un état de la fréquence cardiaque ; et

   un moyen de détermination de l'état de sommeil (102b) pour déterminer, parmi une pluralité de stades de sommeil, celui dans lequel se trouve l'utilisateur ;

   **caractérisé en ce que**

   le moyen de détermination de l'état de sommeil (102b) est configuré pour déterminer le stade de sommeil de manière à distinguer, parmi les stades de sommeil, cinq stades incluant un stade de sommeil paradoxal (REM), et, dans l'ordre du stade de sommeil le plus léger, un premier stade de sommeil non paradoxal N1, un deuxième stade de sommeil non paradoxal N2, un troisième stade de sommeil non paradoxal N3, et un quatrième stade de sommeil non paradoxal N4, dans lequel

   le sommeil léger est classé dans le premier stade de sommeil non paradoxal N1 et le deuxième stade de sommeil non paradoxal N2 ; et

   le moyen de détermination de l'état de sommeil (102b) est configuré pour déterminer dans lequel de la pluralité de stades de sommeil se trouve l'utilisateur à partir de caractéristiques indiquées par une pluralité de paramètres de variabilité de la fréquence cardiaque en utilisant uniquement les paramètres de variabilité de la fréquence cardiaque.

2. Appareil de détermination de l'état de sommeil (100) selon la revendication 1, dans lequel le moyen de détermination de l'état de sommeil (102b) est configuré pour déterminer lequel du premier stade de sommeil non-REM-N1 ou du deuxième stade de sommeil non-REM-N2 les paramètres de variabilité de la fréquence cardiaque de l'utilisateur indiquent, selon une première condition de détermination qui est établie sur la base d'une corrélation entre les paramètres de variabilité de la fréquence cardiaque et le premier stade de sommeil non-REM-N1 et une deuxième condition de détermination qui est établie sur la base d'une corrélation entre les paramètres de variabilité de la fréquence cardiaque et le deuxième stade de sommeil non-REM-N2.

3. Appareil de détermination de l'état de sommeil (100) selon la revendication 1, dans lequel le moyen de détermination de l'état de sommeil (102b) est configuré pour déterminer un stade de sommeil à partir des paramètres de variabilité de la fréquence cardiaque, la détermination étant basée sur des données de résultat d'apprentissage qui sont obtenues par apprentissage automatique, à partir de données d'entraînement pour lesquelles les stades de sommeil sont connus, en utilisant les paramètres de variabilité de la fréquence cardiaque comme entrées.

4. Appareil de détermination de l'état de sommeil (100) selon la revendication 3, dans lequel un algorithme pour l'apprentissage automatique est un réseau neuronal, une machine à vecteurs de support, une forêt aléatoire, un réseau neuronal récurrent ou un modèle de Markov caché.

**5.** Appareil de détermination de l'état de sommeil (100) selon la revendication 1,
dans lequel le moyen de détermination de l'état de sommeil (102b) est configuré pour déterminer dans quel stade de sommeil se trouve l'utilisateur, la détermination étant basée sur une fonction indiquant une probabilité de se trouver dans chacune de la pluralité de stades de sommeil, la fonction ayant les paramètres de variabilité de la fréquence cardiaque comme variables, la pluralité de stades de sommeil incluant le premier stade de sommeil non-REM-N1 et le deuxième stade de sommeil non-REM-N2.

**6.** Procédé de détermination du sommeil exécuté par un ordinateur comprenant :

une étape d'obtention de données de fréquence cardiaque (SB-3) consistant à obtenir, sur la base de données concernant une fréquence cardiaque du corps d'un utilisateur, une composante de très basse fréquence (VLF), un rapport entre une composante de basse fréquence (LF) et une composante de haute fréquence (HF), un intervalle de battement cardiaque (RRI) moyen et un écart type de chaque intervalle de battement cardiaque (RRI) de la fréquence cardiaque en tant que paramètres de variabilité de la fréquence cardiaque indiquant un état de la fréquence cardiaque ; et
une étape de détermination de l'état de sommeil (SB-4) consistant à déterminer, parmi une pluralité de stades de sommeil, celui dans lequel se trouve l'utilisateur ;
**caractérisé en ce que**
dans l'étape de détermination de l'état de sommeil (SB-4), le stade de sommeil est déterminé de manière à distinguer, parmi les stades de sommeil, cinq stades incluant un stade de sommeil paradoxal (REM) et, dans l'ordre du stade de sommeil le plus léger, un premier stade de sommeil non paradoxal N1, un deuxième stade de sommeil non paradoxal N2, un troisième stade de sommeil non paradoxal N3 et un quatrième stade de sommeil non paradoxal N4, dans lequel
le sommeil léger est classé dans le premier stade de sommeil non paradoxal N1 et le deuxième stade de sommeil non paradoxal N2 ;
dans l'étape de détermination de l'état de sommeil (SB-4), il est déterminé dans lequel de la pluralité de stades de sommeil l'utilisateur se trouve à partir de caractéristiques indiquées par une pluralité de paramètres de variabilité de la fréquence cardiaque en utilisant uniquement les paramètres de variabilité de la fréquence cardiaque.

**7.** Programme de détermination du sommeil pour amener un ordinateur à réaliser :

une fonction d'obtention de données de fréquence cardiaque consistant à obtenir, sur la base de données concernant une fréquence cardiaque du corps d'un utilisateur, une composante de très basse fréquence (VLF), un rapport entre une composante de basse fréquence (LF) et une composante de haute fréquence (HF), un intervalle de battement cardiaque (RRI) moyen et un écart type de chaque intervalle de battement cardiaque (RRI) de la fréquence cardiaque en tant que paramètres de variabilité de la fréquence cardiaque indiquant un état de la fréquence cardiaque ; et
une fonction de détermination de l'état de sommeil consistant à déterminer, parmi une pluralité de stades de sommeil, celui dans lequel se trouve l'utilisateur ;
**caractérisé en ce que**
la fonction de détermination de l'état de sommeil détermine le stade de sommeil de manière à distinguer, parmi les stades de sommeil, cinq stades incluant un stade de sommeil paradoxal (REM) et, dans l'ordre du stade de sommeil le plus léger, un premier stade de sommeil non paradoxal N1, un deuxième stade de sommeil non paradoxal N2, un troisième stade de sommeil non paradoxal N3 et un quatrième stade de sommeil non paradoxal N4, dans lequel
le sommeil léger est classé dans le premier stade de sommeil non paradoxal N1 et le deuxième stade de sommeil non paradoxal N2 ;
la fonction de détermination de l'état de sommeil détermine dans lequel de la pluralité de stades de sommeil l'utilisateur se trouve à partir de caractéristiques indiquées par une pluralité de paramètres de variabilité de la fréquence cardiaque en utilisant uniquement les paramètres de variabilité de la fréquence cardiaque.

# FIG. 1

# FIG. 2

```
                    ┌─────────────────┐
                    │      START      │
                    └─────────────────┘
                            │
                            ▼
        ┌───────────────────────────────┐
        │      SET A SLEEP STAGE         │ ⌇ SB-1
        │   DETERMINATION TECHNIQUE      │
        └───────────────────────────────┘
                            │
                            ▼
        ┌───────────────────────────────┐
        │      STORE THE HEART RATE      │ ⌇ SB-2
        │         SIGNAL DATA            │
        └───────────────────────────────┘
                            │
                            ▼
        ┌───────────────────────────────┐
        │      EXTRACT HEART RATE        │ ⌇ SB-3
        │   VARIABILITY PARAMETERS       │
        └───────────────────────────────┘
                            │
                            ▼
        ┌───────────────────────────────┐
        │     DETERMINE A SLEEP STAGE    │ ⌇ SB-4
        └───────────────────────────────┘
                            │
                            ▼
                    ┌─────────────────┐
                    │       END       │
                    └─────────────────┘
```

# FIG. 3

# FIG. 4

# FIG. 5

# FIG. 6

# FIG. 7

# FIG. 8

INPUT LAYER                 INTERMEDIATE
                            LAYER                    OUTPUT LAYER

# FIG. 9

# FIG. 10

# FIG. 11

# FIG. 12

# FIG. 13

# FIG. 14

# FIG. 15

## FIG. 16

## FIG. 17

# FIG. 18

# FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015190086 A1
- US 2016310696 A1

**Non-patent literature cited in the description**

- **SM ISA** ; **I WASITO**. Kernel dimensionality reduction on sleep stage classification using ECG signal. *IJCSI International Journal of Computer Science Issues*, July 2011, vol. 8 (4)
- **K TANIDA** ; **M SHIBATA**. Sleep stage assessment using power spectral indices of heart rate variability with a simple algorithm: limitations clarified from preliminary study. *Biological Research for Nursing*, 2013, vol. 15 (3)
- **P FONSECA** ; **X LONG** ; **M RADHA** ; **R HAAKMA** ; **R AARTS** ; **J ROLINK**. Sleep stage classification with ECG and respiratory effort. *Physiological Measurement*, vol. 36 (10)
- **SHANSHAN YU et al.** Automatic sleep stage classification based on ECG and EEG features for day time short nap evaluation. *10th World Congress on Intelligent Control and Automation (WCICA)*, 06 July 2020, 4974-4977
- **HIDETO HIRASAWA** ; **YOSHIKATA ATSUMI**. Polysomnographic Changes in Normal Human Aging. *Japanese Journal of Geriatrics*, June 1997, vol. 34 (6)
- **MD BASUNIA et al.** Relationship of symptoms with sleep-stage abnormalities in obstructive sleep apnea-hypopnea syndrome. *J Community Hosp Intern Med Perspect*, 2016, vol. 6 (4), 32170
- **DENIS MARTINEZ et al.** Dimensions of sleepiness and their correlations with sleep-disordered breathing in mild sleep apnea. *J. bras. pneumol.*, June 2009, vol. 35 (6)
- **M. DUMONT** ; **F. JURYSTA** ; **J. LANQUART** ; **P. MIGEOTTE** ; **P. BORNE** ; **P. LINKOWSKI**. Interdependency between heart rate variability and sleep EEG: linear/non-linear?. *Clinical Neurophysiology*, 2004, vol. 115 (9), 2031-2040
- **J.R. YEH** ; **C.K. PENG** ; **M.T. LO** ; **C.H. YEH** ; **S.C. CHEN** ; **C.Y. WANG** ; **P.L. LEE** ; **J.H. KANG**. Investigating the interaction between heart rate variability and sleep EEG using nonlinear algorithms. *Journal of Neuroscience Methods*, 2013, vol. 219 (2), 233-239
- **H. AKAIKE**. Fitting autoregressive models for prediction. *Annals of the Institute of Statistical Mathematics*, 1969, vol. 21 (1), 243-247
- **T. KOHONEN**. Self-organizing formation of topologically correct feature maps. *Biological Cybernetics*, 1982, vol. 43, 59-69
- **A.J. VITERBI**. Error bounds for convolutional codes and an asymptotically optimum decoding algorithm. *IEEE Transactions on Information Theory*, 1967, vol. 13 (2), 260-269
- **T. WILLEMEN** ; **D.V. DEUN** ; **V. VERHAERT** ; **M. VANDEKERCKHOVE** ; **V. EXADAKTYLOS** ; **J. VERBRAECKEN** ; **S.V. HUFFEL** ; **B. HAEX** ; **J.V. SLOTEN**. An evaluation of cardiorespiratory and movement features with respect to sleep-stage classification. *IEEE Journal of Biomedical and Health Informatics*, 2014, vol. 18 (2), 661-669
- **DENIS MARTINEZ** ; **MAGALI SANTOS LUMERTZ** ; **MARIA DO CARMO SFREDDO LENZ**. Dimensions of sleepiness and their correlations with sleep-disordered breathing in mild sleep apnea. *J Bras Pneumol.*, 2009, vol. 35 (6), 507-514